# EUROPEAN PATENT SPECIFICATION

(11) **EP 3 585 893 B1**
(45) Date of publication and mention of the grant of the patent: **13.10.2021**
(21) Application number: 18714073.6
(22) Date of filing: 21.02.2018
(51) Int. Cl.: C12N 15/10, C12Q 1/683, C12N 15/64, C12N 15/66

(54) **METHOD FOR ASSEMBLY OF POLYNUCLEIC ACID SEQUENCES USING PHOSPHOROTHIOATE BONDS WITHIN LINKER OLIGOS**
VERFAHREN ZUR ASSEMBLIERUNG VON POLYNUKLEINSÄURESEQUENZEN UNTER VERWENDUNG VON PHOSPHORTHIOATBINDUNGEN IN LINKER OLIGOS
PROCÉDÉ D'ASSEMBLAGE DE SÉQUENCES DE POLY(ACIDE NUCLÉIQUE) EN UTILISANT DES LIAISONS PHOSPHOROTHIOATES À L'INTÉRIEUR D'OLIGOMÈRES LIEURS

(30) Priority: 22.02.2017 US 201762461938 P
(43) Date of publication of application: 01.01.2020
(73) Proprietor: Ingenza Ltd., Roslin BioCentre Midlothian EH25 9PP (GB)
(72) Inventor: MCCOLM, Stephen, Edinburgh, EH7 4HA (GB)
(74) Representative: HGF
(86) International application number: PCT/IB2018/000214
(87) International publication number: WO 2018/154384

(56) References cited:
- EP-A1- 2 395 087
- WO-A1-2009/023676
- WO-A1-2010/070295
- WO-A2-03/027258
- WO-A2-2009/103027
- WO-A2-2011/161413
- LU CHENFENG ET AL: "Comparison of multiple gene assembly methods for metabolic engineering", APPLIED BIOCHEMISTRY AND BIOTECHNOLOGY ; PART A: ENZYME ENGINEERING AND BIOTECHNOLOGY, HUMANA PRESS INC, NEW YORK, vol. 137, 1 April 2007 (2007-04-01), pages 703-710, XP002569290, ISSN: 0273-2289
- RESHMA P SHETTY ET AL: "Engineering BioBrick vectors from BioBrick parts", JOURNAL OF BIOLOGICAL ENGINEERING, vol. 2, no. 1, 1 January 2008 (2008-01-01) , page 5, XP055065521, ISSN: 1754-1611, DOI: 10.1186/1754-1611-2-5 cited in the application
- MARKO STORCH ET AL: "BASIC: A New Biopart Assembly Standard for Idempotent Cloning Provides Accurate, Single-Tier DNA Assembly for Synthetic Biology", ACS SYNTHETIC BIOLOGY, vol. 4, no. 7, 17 July 2015 (2015-07-17), pages 781-787, XP055343133, Washington, DC,USA ISSN: 2161-5063, DOI: 10.1021/sb500356d

## Description

### Cross Reference to Related Application

The present application claims priority to U.S. Provisional Patent Application No. 62/461,938 filed February 22, 2017.

### Background

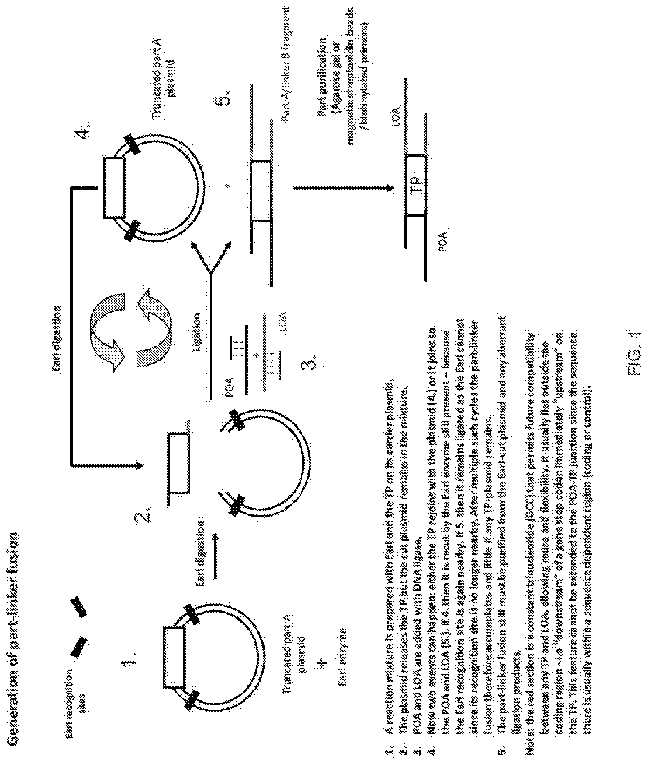

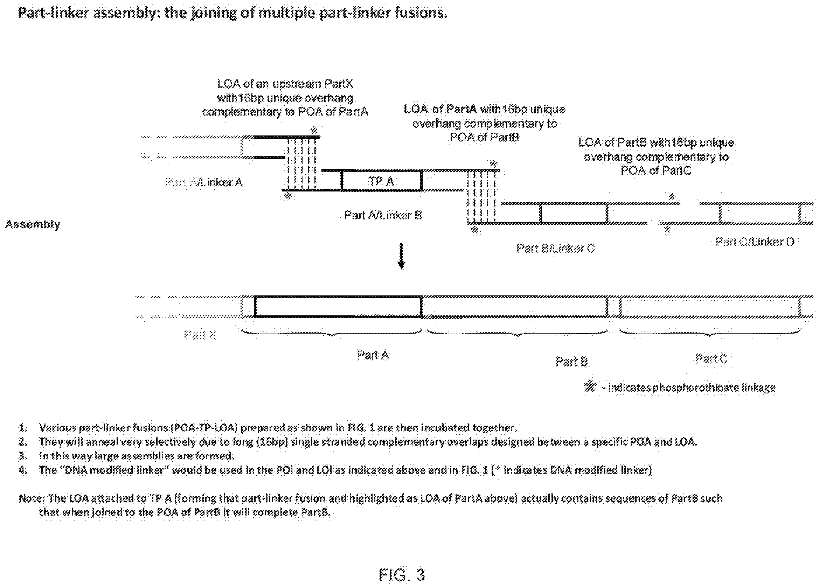

The present invention is generally related to the field of synthetic biology. More particularly, the present invention relates to a method of combinatorial polynucleic acid assembly that introduces phosphorothioate bonds into the linker oligos during the assembly procedure in order to use exonucleases to isolate the DNA of interest, thereby eliminating any cumbersome purification steps, such as gel electrophoresis and significantly increasing the overall selectivity and efficiency of the method. The present invention improves on the polynucleic acid assembly method described in U.S. Patent No. 8,999,679 (also referred to herein as inABLE^{®} assembly or inABLE^{®} technology) which is incorporated by reference. inABLE^{®} assembly generally takes DNA truncated parts (TPs), such as genes, from a vector using type IIs restriction enzymes, and ligates small DNA "linkers" to the ends of the truncated parts forming part-linker fusions, as shown in FIG. 1. The part-oligo annealed "POA" linker at the end of one part-linker fusion is complementary to the linker-oligo annealed "LOA" linker at the end of a second part-linker fusion. In this way, the part-linker fusions can self-assemble with high efficiency into a single piece of DNA in a predetermined order as shown in FIG. 3.

The present invention introduces a phosphorothioate bond by replacing a non-bridging oxygen within the phosphate backbone of the nucleic acid sequence with a sulfur (S) atom. Introduction of this sulfur atom results in an internucleotide linkage that is resistant to nuclease cleavage. Consequently, by adding such modified S linkers to form nuclease resistant ends of part-linker joined DNA fragments/fusions, the present invention allows the use of exonucleases to degrade parts that do not have linkers ligated to their ends to isolate only the part-linker DNA of interest and avoiding the need for purification by gel electrophoresis.

Molecular cloning using endonucleases and DNA ligase has been employed for the last 40 years. It relies on a "cutting and pasting" methodology in which DNA is cleaved at specific sites with restriction enzymes to generate overhangs, typically 3-4 nucleotides in length. The DNA fragments are then mixed, and the compatible single stranded DNA overhangs anneal before being covalently joined by a DNA ligase. Typically, two or three fragments of DNA are combined using this approach. The field of synthetic biology however is built around the high throughput combination of standardized DNA fragments to generate genetic pathways for the production of chemicals, fuels, foods, pharmaceuticals and other products. Therefore, the construction of complex DNA pathways and associated regulatory regions is not practical using the known hierarchical techniques available. This limitation led to the development of multiple DNA assembly techniques for the construction of complex DNA vectors in a rapid and predictable manner.

BioBricks (Shetty, R.P., D. Endy, and T.F. Knight, Jr., Engineering BioBrick vectors from BioBrickparts. J Biol Eng, 2008. 2: p. 5) was the first DNA assembly technique to gain traction amongst the scientific community. BioBricks are standardized fragments of DNA which can be joined together in a Lego^{®}-like fashion. The BioBrick approach allows for the combination of up to three BioBricks with the resulting construct becoming a new BioBrick, thus allowing subsequent rounds of DNA assembly. Both the assembly method and parts are standardized in the sense that the enzymes used are constant and therefore the 5' and 3' terminal sequences are constant. The main limitations of this approach are the time-consuming construction of complex vectors due to the stepwise nature of the assembly process, the sequence dependent nature of the approach, the associated introduction of additional non-relevant DNA and the vast diversity of DNA sequences, which greatly limits the ability to recycle the standardized BioBricks.

Gibson, *et al* (Gibson, D.G., et al., Enzymatic assembly of DNA molecules up to several hundred kilobases. Nat Meth, 2009. 6(5): p. 343-345) describes an isothermal method for the enzymatic assembly of multiple fragments of DNA. This technique involves the one pot assembly of DNA fragments through the combination of linear DNA parts with at least 25 base pairs of homology, a 5' exonuclease, a DNA polymerase and a DNA ligase. Initially, the exonuclease degrades the linear parts in a 5' to 3' direction resulting in the generation of single stranded overhangs enabling homologous regions to anneal to each other. Next, a high-fidelity DNA polymerase fills in any gaps before the nicks in the final construct are sealed by a DNA ligase. The advantages of this technique over BioBricks is the increased number of parts which can be combined, the fact it is largely sequence independent because no specific restriction sites are required to be omitted from part sequences, and that it results in an assembly that does not have to include additional unnecessary nucleotides as a consequence of the method itself. However, the Gibson method has several disadvantages, including the fact that DNA fragments must be prepared *de novo* for each assembly, the parts must be at least 250 base pairs, repeated sequences are not tolerated, and the use of a DNA polymerase may impact reliability.

A number of the issues encountered with the Gibson DNA assembly method are addressed by the Golden Gate DNA assembly methodology (Engler, C., R. Kandzia, and S. Marillonnet, A One Pot, One Step, Precision Cloning Method with High Throughput Capability. PLoS ONE, 2008. 3(11): p. e3647). This technique relies on type IIs endonucleases which are restriction enzymes that cut distal from their recognition site. Although a number of type IIs endonucleases are available, BsaI is commonly used for Golden Gate DNA assembly. Parts to be assembled using Golden Gate assembly are amplified and flanked by appropriate recognition sites. The polymerase chain reaction ("PCR") products are mixed with a type IIs endonuclease and DNA ligase in a one pot reaction, an initial stage of digestion results in the part being liberated from the flanking restriction sites resulting in 4 base single stranded DNA overhangs that facilitate ligation between the parts containing complementary overhangs. Cycles of digestion and ligation are used to reduce re-ligation of original fragments and thereby enrich the product of interest in the mixture. Compared to Gibson assembly, this technique is less sequence independent as it requires the parts to be void of Bsal recognition sites. However, the Golden Gate method is limited because it results in overhangs of only four nucleotides in length. With a desire to increase selectivity by having at least two nucleotide differences between each set of overhangs, in complex assemblies it may not be possible to find specific overhangs to ensure the efficient assembly of a given fragment using the Golden Gate method.

At present, gel electrophoresis is the standard method to purify desired DNA from a mixture. This approach utilizes an electric field to migrate DNA through a gel containing small pores. The fragments of DNA travel through the gel at a speed inversely proportionate to their length. Therefore, smaller fragments travel further on the gel and DNA of the desired size can be identified through comparison with a co-electrophoresed set of DNA fragments of a known size. The desired DNA is subsequently retrieved from the gel through manual excision of the gel fragment and extraction of DNA from the gel matrix using commercially available kits (i.e., QIAquick gel extraction ―QIAGEN Corp.). This process results in low throughput and creates a bottleneck in the DNA assembly process. For instance, a limited number of samples can be loaded per gel with each gel taking approximately 30 minutes to setup and 30 minutes at a minimum to complete electrophoresis to achieve an acceptable separation of DNA fragments, while the extraction of the DNA adds an additional 30 minutes per gel. Furthermore, this is a manual process not amenable to automation. An additional limitation to this approach is the resolution that can be achieved through gel electrophoresis. The DNA of interest contains 5' and 3' linkers which constitute an increase in size of ∼ 50 base pair, over a DNA part which lacks either the 5' and/or or the 3' linker. The part itself can often be ≥ 1000 base pairs in length so this change in size may be only ≤ 5% and thereby indistinguishable by gel electrophoresis. As a result, DNA lacking one (or both) of the linkers can readily be co-extracted and carried through into the assembly stage where its presence is detrimental to the assembly process.

To date, attempts to address the setbacks discussed above have largely focused on the utilization of biotinylated linkers and streptavidin beads. The technique relies on the high affinity with which biotin binds to streptavidin. This approach improves throughput and is in theory specific for purification of DNA which contains both 5' and 3' linkers. Biotinylated primers are available from all major primer manufacturers while small scale spin columns packed with streptavidin beads are produced by a number of companies.

U.S. Patent No. 8,999,679, titled "Method for Assembly of Polynucleic Acid Sequences," focuses on the combinatorial assembly of DNA fragments such as genes and regulatory regions for the construction of complex genetic pathways. This technology is also referred to herein as inABLE^{®} assembly and/or inABLE^{®} technology. inABLE^{®} assembly combines the advantages of the Gibson assembly (long, single-stranded homologous overhangs) and the Golden Gate assembly (no DNA polymerase needed, a tolerance of repeated sequences, and no limit on DNA length). The technique described in the '679 patent relies on initial cloning of a 5' truncated version of the DNA fragment (referred to as a truncated part) of interest flanked by type IIs restriction sites (typically SapI or EarI). Cycles of digestion and ligation are utilized to initially cleave the truncated part from its cloning vector, prior to annealing linkers to the 5' and 3' of the truncated part (referred to as creation of a part-linker fusion). The ligation of these linkers generates 16 nucleotide single stranded DNA overhangs at each end of the part-linker fusion which provide extensive complementarity between the parts which are to be assembled. In the final stage, multiple part-linker fusions are mixed and assembled in an order specified through the unique complementarity of individual pairs of 16 nucleotide overhangs. Annealing linkers with 16 nucleotide single stranded extensions increases the complementarity between parts (as opposed to short 4 nucleotide single stranded overhangs created by traditional digestion and ligation techniques). The increased length of these extensions greatly increases the selectivity of the process, permitting multiple fragments of DNA to be assembled more efficiently using the inABLE^{®} assembly, whereas traditional cloning typically allows the efficient ligation of only 2-3 fragments at most.

Biotinylated oligos (referred to as purification oligos) complementary to the 16-nucleotide single stranded overhangs of inABLE^{®} parts can be ordered. However, it is not feasible to confirm whether both 5' and 3' linkers have been attached to a part. For instance, presence of only one linker would still result in the DNA part-linker binding to the streptavidin. In order to purify the desired DNA fragment, the 3' linker must first be ligated, this fragment of DNA purified, and the residual purification oligo removed before the process is repeated for the 5' linker. This adds additional steps to the process increasing the time to assemble the required vector, an extra cost to purchase the additional purification oligo, the need for two stages of purification, and the unavoidable loss of product at each stage. This results in a less efficient, albeit somewhat higher throughput process than what is achievable with gel extraction-based purification.

A major setback with the technology described in the '679 patent is the requirement to purify part-linker fusions through methods such as gel electrophoresis or biotinylated primers and streptavidin beads. Both of these purification approaches have limitations and present a bottleneck in the current workflow. The running of agarose gels and excision of the DNA of interest is a cumbersome, low throughput approach with the added disadvantage that the DNA extracted from the gel is likely a combination of the desired part-linker fusion and fragments that lack one or both linkers, which, along with the residual vector DNA, greatly reduce the efficiency of the DNA assembly stage. The '679 patent describes utilization of biotinylated primers and streptavidin beads as a potential solution to these concerns. However, as discussed above, this approach requires additional complex stages of processing which increase the length of time by up to six hours and reduce the overall efficiency to construct the vector of interest. To address this setback, the present invention combines the use of phosphorothioate bonds coupled to exonuclease treatment as means to purify DNA within a DNA assembly workflow.

Exonucleases are enzymes which degrade DNA in a stepwise manner from the end of the polynucleotide chain in either a 5' → 3' or 3' → 5' direction. Phosphorothioate bonds are generated through replacing a non-bridging oxygen within the phosphate backbone with a sulfur atom. The introduction of the sulfur atom renders the internucleotide linkage resistant to endo- and exonuclease cleavage. This property can be used to protect DNA of interest from exonuclease attack. Through the introduction of phosphorothioate bonds within the 5' and 3' linkers, only DNA which has linkers annealed at both ends is protected, while the remaining DNA is degraded through exonuclease treatment. This offers specificity which cannot be rivalled by gel electrophoresis (due to the resolution which can be achieved) and biotin/streptavidin purification (a separation with potential for contamination by non-specifically bound DNA or a part with a linker attached at only one end). The process does not involve additional steps during ligation of linkers to parts and replaces the gel electrophoresis stage with the addition of an exonuclease and incubation for 30 minutes or less. This enzyme addition and incubation has the potential for extremely high throughput and automation, unlike gel electrophoresis.

Thus, the present invention addresses the drawbacks of the prior assembly methods by providing a highly selective, efficient and high throughput DNA assembly approach that is easily automated through the use of a liquid handling robot and isolates only the DNA of interest, while degrading the remaining DNA in the reactions. Specifically, the present invention addresses the need for a purification method which matches the specificity and high throughput nature of biotin/streptavidin while reducing the number of steps and overcoming the inefficiencies of gel electrophoresis. The potential advantages of implementing a phosphorothioate/exonuclease approach are discussed below.

### Summary of The Invention

The present invention describes an improved DNA assembly method having high throughput which can be readily automated to prepare complex polynucleic acid sequences with increased efficiency. The method of assembly disclosed herein describes a novel DNA purification technique that utilizes phosphorothioate linkers and exonucleases to isolate the DNA of interest.

Generally, a method for assembling polynucleic acids is described providing at least two DNA truncated parts. A DNA modified linker is ligated to both ends of each DNA truncated part to form at least two part-linker fusions. The DNA modified linker has an internucleotide modification that is resistant to nuclease cleavage. The part-linker fusions are isolated using an exonuclease and the part-linker fusions are joined to form a polynucleic acid sequence.

In one embodiment, the present invention introduces between 1 and 3 phosphorothioate linkages at the 3' ends of both a part-oligo long (POl) and a linker oligo long (LOl) oligonucleotide, which are then annealed respectively with their complementary part-oligo short (POs) and linker-oligo short (LOs) oligonucleotides to generate a part-oligo annealed (POA) and a linker-oligo annealed (LOA), as shown in FIG. 1. The POA and LOA are then ligated to a given part, (cleaved from its cloning vector using SapI or EarI) resulting in a part-linker fusion which is resistant to cleavage by DNA exonucleases. Significantly, the cloning vector which originally carried the truncated part, prior to SapI/EarI digestion as well as any aberrant part-linker fusions (i.e., missing either POA and /or LOA) are not protected from digestion. This method can be utilized to purify part-linker fusions from contaminating DNA through treatment with an exonuclease.

The utilization of phosphorothioate linkers in the present invention increases the efficiency of the DNA assembly process greatly reduces the length of time required to build the required constructs, increases the robustness of the procedure and permits automation of the process. A number of these advantages are described in more detail below.

### Brief Description of The Drawings

The present invention is described by way of reference to the following figures and examples which are provided for the purpose of illustration only and are not to be construed as limiting on the invention.
FIG. 1 is a schematic of the generation of a part-linker fusion. FIG. 1 depicts cleavage of truncated part from vector using type IIs restriction enzyme (typically Sapl or EarI), followed by ligation of POA and LOA linkers to generate a part-linker fusion.

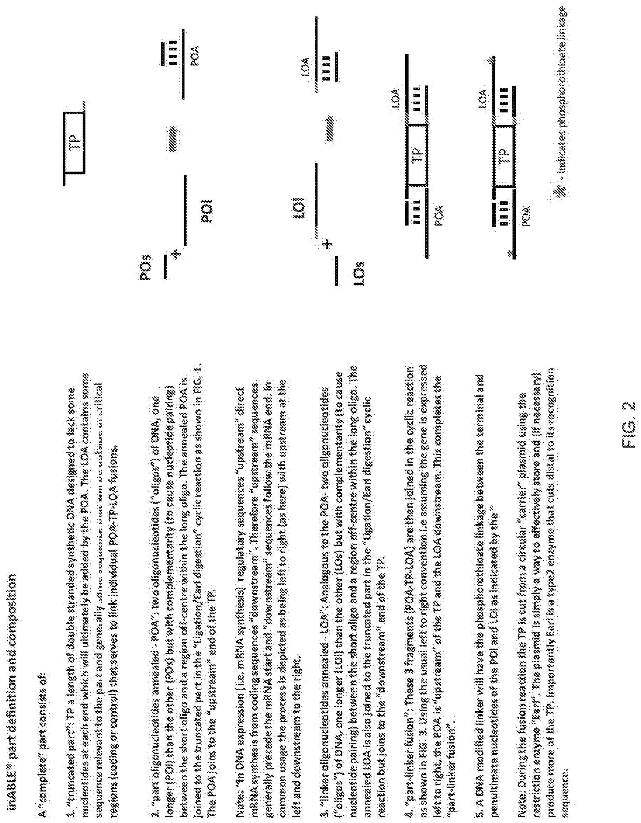

FIG. 2 is a detailed schematic of a complete part-linker fusion of the present invention.
FIG. 3 is a schematic of DNA assembly part-linker fusions using complementary overhangs between part-linker fusions.

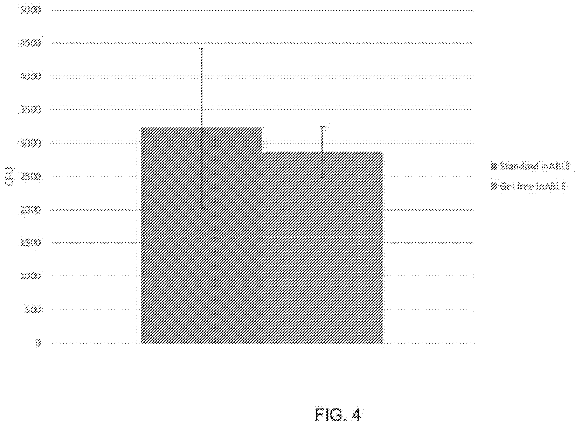

FIG. 4 compares the effect of coupled phosphorothioate bonds and Exonuclease III (Exo III) treatment on assembly efficiency for more complex assemblies. Five DNA fragments were assembled, four of which confer resistance to an antibiotic and one an *E. coli* origin of replication. This allows for selection of cells containing the expected assembly on media supplemented with four antibiotics. The assembly efficiency is calculated by counting the colonies.

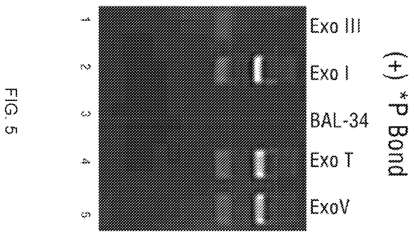

FIG. 5 is a part-linker fusion reaction analyzed via agarose gel following treatment with various exonucleases. Lane 1 highlights treatment with Exonuclease III (Exo III) which removes the contaminating DNA (higher molecular weight band seen in lanes 2, 4 and 5) while not digesting the phosphorothioate protected part-linker fusion (lower band). The other exonucleases explored either did not remove the contaminating DNA (lanes 2, 4 and 5) or digested all DNA present (lane 3).

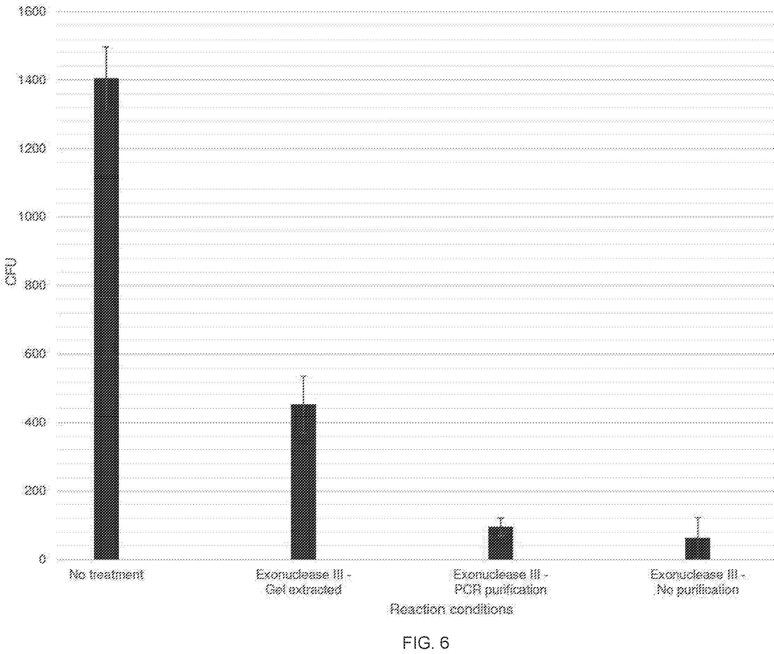

FIG. 6 reports the effect of exonuclease treatment on the assembly efficiency of two fragments of DNA that contain no phosphorothioate bonds. Each fragment conferred resistance to a different antibiotic, allowing for selection of cells harboring the expected assembly on media supplemented with both antibiotics. Counting the number of colonies allows for the assembly efficiency to be calculated. In this experiment the standard inABLE procedure yielded ~ 1400 colonies carrying the expected assembly. When treatment with Exo III was used followed by purification via agarose gel this number dropped to ∼450 colonies, a decrease in transformation efficiency of 68 %. The efficiency further decreases when the exonuclease treatment is followed by a PCR clean up and no purification (∼93% decrease).

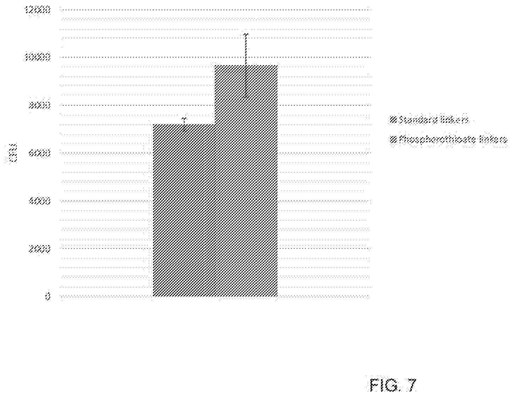

FIG. 7 compares the effect of phosphorothioate bonds on assembly efficiency. Five DNA fragments assembled, four of which confer resistance to an antibiotic and one an *E*. *coli* origin of replication. This allows for selection of cells containing the expected assembly on media supplemented with four antibiotics. Through the counting of colonies, the assembly efficiency is calculated.

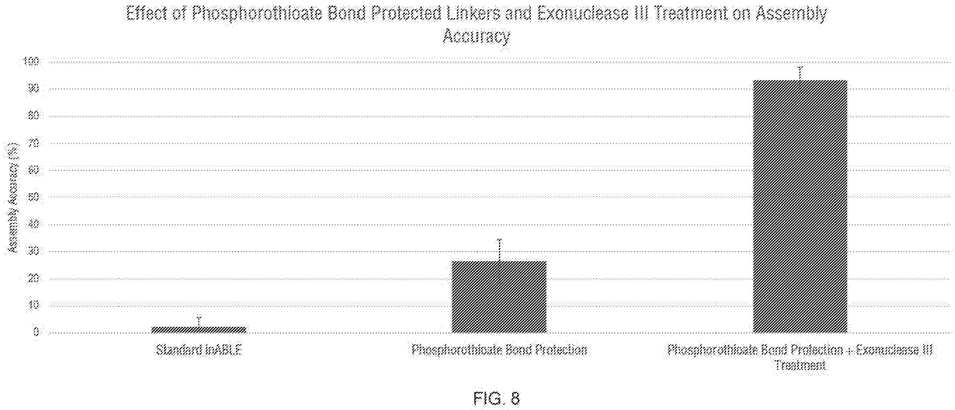

FIG. 8 shows the effect of phosphorothioate bond protected linkers and exonuclease III treatment on assembly accuracy.

### Detailed Description

The present invention is capable of embodiments in many different forms. Preferred embodiments of the invention are disclosed with the understanding that the present disclosure is to be considered an exemplification of the principles of the invention and are not intended to limit the broad aspects of the invention to the embodiments illustrated.

As discussed above, U.S. Patent No. 8,999,679 discloses assembly of DNA fragments for the construction of complex genetic pathways by cleaving DNA truncated parts, such as genes, regulators, markers, reporters, etc. from a vector using type IIs restriction enzymes and ligating small DNA "linkers" to ends of the truncated parts forming part-linker fusions. The '679 patent details the assembly of DNA parts through the use of linkers in a one pot reaction. FIG. 1 depicts the part-linker fusion reaction of the '679 patent, in which the linkers are annealed and then ligated to the truncated part through cycles of digestion and ligation.

Referring to FIG. 1, a reaction mixture is prepared with EarI and the truncated part (TP) on its carrier plasmid. The plasmid releases the TP but the cut plasmid remains in the mixture. POA and LOA are then added with DNA ligase. At this point, two things can occur: 1) either the TP rejoins with the plasmid (as shown in 4) or it joins to the POA and LOA (as shown in 5). If the TP rejoins the plasmid, then it is recut by the EarI enzyme still present - because the EarI recognition site is again nearby. If the TP joins to the POA and LOA, then it remains ligated as the EarI cannot cut since its recognition site is no longer nearby. After multiple such cycles the part-link fusion accumulates and little if any TP-plasmid remains. The part-linker fusion still must be purified from the EarI-cut plasmid and any aberrant ligation products.

FIG. 2 illustrates the assembly of a complete part-linker of the present invention. A TP of double stranded synthetic DNA is provided. The TP is designed to lack a number of nucleotides at the 5'end that will ultimately form the POA and LOA sequence. POA refers to two oligonucleotides ("oligos") of DNA, one longer (POl) than the other (POs) but with complementarity (to cause nucleotide pairing) between the short oligo and a region off-center within the long oligo. The annealed POA is joined to the truncated part in the "Ligation/EarI digestion" cyclic reaction shown in FIG. 1. The POA joins to the "upstream" end of the TP. It should be noted, in DNA expression (i.e. mRNA synthesis) regulatory sequences "upstream" direct mRNA synthesis from coding sequences "downstream". Therefore "upstream" sequences generally precede the mRNA start and "downstream" sequences follow the mRNA end. In common usage the process is depicted as being left to right (as here) with upstream at the left and downstream to the right.

LOA is analogous to the POA, and refers to two oligos of DNA, one longer (LOl) than the other (LOs) but with complementarity (to cause nucleotide pairing) between the short oligo and a region off-center within the long oligo. The annealed LOA is also joined to the truncated part in the "Ligation/Earl digestion" cyclic reaction as shown in FIG. 1, but joins to the "downstream" end of the TP. The part-linker fusion refers to the three fragments (POA-TP-LOA) which are then joined in the cyclic reaction shown in FIG. 3. Using the usual left to right convention (i.e. assuming the gene is expressed left to right) the POA is "upstream" of the TP and the LOA downstream. This forms the complete the "part-linker fusion." Significantly, the present invention introduces a DNA modified linker having a phosphorothioate linkage between the terminal and penultimate nucleotides of the POl and LOl as indicated by the ^{∗} in FIG. 2.

FIG. 3 depicts the assembly of individual part-linker fusions which can anneal and assemble using the 16-nucleotide single stranded extensions. Since the linker at the end of one part-linker fusion is complementary to the "linker" at one end of the second part-linker fusion, the part-linker fusions can self-assemble selectively into one piece of DNA in a predetermined order, as shown in FIG. 3. The POA and LOA linkers contain ∼16 nucleotide single stranded overhangs which provide complementarity between the fragments of DNA. Overhangs are then utilized to assemble the fragments of DNA in a predefined order with efficiency not achievable with standard molecular cloning.

The present invention provides improvements over the '679 patent. While the part-linker fusion reaction (or inABLE^{®} assembly) remains the same, the linkers of the present invention are modified to include phosphorothioate bonds, as shown in FIGS. 2 and 3. Referring to FIG. 3, part-linker assembly is achieved by joining multiple part-linker fusions. First, various part-linker fusions (POA-TP-LOA) are prepared as shown in FIG. 1. The part-linker fusions are then incubated together. During incubation, the part-linker fusions will anneal very selectively due to long (16bp) single stranded complementary overlaps that are designed between a specific POA and LOA. As a result, large assemblies are formed. An internucleotide modification resistant to nuclease cleavage is made in the POl and LOl as shown in FIGS. 2 and 3. In one embodiment the internucleotide modification is a phosphorothioate bond.

The presence of these phosphorothioate bonds are utilized to purify the part-linker DNA using an exonuclease treatment that yields additional benefits not known in the art. The use of this technique highlights the requirement to purify the DNA fragments after ligation of the linkers to a given part, which is the key bottleneck in the technique. Specifically, the present invention provides a DNA purification method that results in greater process efficiency through higher throughput and specificity in reduced time, while permitting automation of the entire process by eliminating the need for gel electrophoresis.

During the process of constructing a DNA vector using inABLE^{®} assembly, each part requires a part-linker fusion step. Automation of the part-linker fusion reaction can be achieved through the use of a liquid handling robot. However, prior to assembly, each part-linker fusion must be purified via gel electrophoresis, which is a cumbersome rate limiting step that results in significant residual quantities of contaminating DNA fragments (non-ligated or partially ligated part and linker). The requirement to run a gel to purify the part-linker fusion limits the compatibility of the inABLE^{®} assembly with automation and the purity of the desired part-linker fusion.

Exonucleases are enzymes which degrade DNA in a stepwise manner from the end of the polynucleotide chain. Exonucleases are enzymes which cleave nucleotides one at a time from the end of a polynucleotide chain in either a 5' → 3' or 3' → 5' direction. The introduction of a phosphorothioate bond renders the internucleotide linkage resistant to exonuclease cleavage. This property can be used to protect DNA of interest from exonuclease attack. Through the introduction of phosphorothioate bonds within the 5' and 3' linkers, only DNA which has linkers annealed is protected, while the remaining DNA is degraded through exonuclease treatment. This offers specificity which cannot be rivalled by gel electrophoresis (due to the resolution which can be achieved) and biotin/streptavidin purification (a separation with potential for contamination by non-specifically bound DNA or a part with a linker attached at only one end). The process does not involve additional steps during ligation of linkers to parts and replaces the gel electrophoresis stage with the addition of an exonuclease and incubation for 30 minutes or less. This enzyme addition and incubation has the potential for extremely high throughput and automation, unlike gel electrophoresis.

The present invention combines the use of phosphorothioate bonds and exonucleases as a means to purify DNA within a DNA assembly workflow. The present invention forms a phosphorothioate bond by replacing a non-bridging oxygen within the phosphate backbone with a sulfur atom during the part-linker fusion step as shown in FIGS. 2 and 3. In one embodiment, the present invention introduces between at least 1 and 3 phosphorothioate linkages at the 3' end of the POl and LOl oligonucleotide (annealed with POs and LOs to generate POA and LOA respectively), as shown in FIG. 3, resulting in part-linker fusions which are resistant to cleavage by exonucleases. Such phosphorothioate containing linkers are known in the art and may be purchased with the S-modification at low cost from major suppliers such as Sigma Aldrich or Integrated DNA Technologies. Significantly, the vector which carried the truncated part prior to SapI/EarI digestion and any aberrant part-linkers (i.e. missing either POA and /or LOA) are not protected. Thus, the present invention can be utilized to purify part-linker fusions from all contaminating DNA which could be present following other purification methods, through treatment with an exonuclease.

The replacement of the sulfur atom renders the internucleotide linkage resistant to nuclease cleavage. While the present invention discloses the use of phosphorothioate bonds, it should be understood that any internucleotide modification which inhibits nuclease cleavage (exonucleases or endonucleases) could be used with the present invention. Through the introduction of an exonuclease treatment, coupled with the introduction of phosphorothioate bonds within the POA and LOA sequences, the present invention discloses a method of purifying the part-linker fusion resulting from the inABLE^{®} assembly without the need to conduct gel electrophoresis. FIG. 1 details the steps of the inABLE^{®} assembly method - ligation of linkers to truncated part through cycles of digestion and ligation. The present invention eliminates the need for the "part purification (agarose gel)" step. Instead, the present invention allows for part purification with the use of exonucleases. Since the exonuclease is unable to cleave phosphorothioate bonds, the part-linker fusion (POA:TP:LOA) is protected from the exonuclease, while the remaining DNA in the reaction, including the vector backbone and aberrant part-linkers (i.e. missing either POA and/or LOA) are digested by the exonuclease. Exonucleases are known in the art and are available for purchase by any provider such as NEB or Thermo. It is important the exonuclease used with the present invention is phosphorothioate sensitive, such as Exo III. While the present invention uses exonucleases active in the 3' to 5' direction, exonucleases which are active in the 5' to 3' direction may similarly be utilized in circumstances where 5' single stranded overhangs are utilized in place of 3' single stranded overhangs

Thus, the present invention provides a technique amenable to automation and results in an increase in the overall assembly reaction efficiency and assembly accuracy. For instance, when the "part-linker fusion" band is excised from a gel it is likely that within the DNA extracted there will be a percentage of aberrant part-linker fusions (i.e. TP+POA bound but not LOA or vice versa). The introduction of the exonuclease treatment will remove these fragments, thereby resulting in much greater overall DNA assembly efficiency.

The DNA purification approach of the present invention may not be suitable for other DNA assembly techniques disclosed in the prior art, such as the Golden Gate and Gibson assemblies, since they both rely on the use of either PCR products or cloned DNA fragments, in which phosphorothioate bonds cannot be introduced. However, it is particularly suitable for DNA assembly using the inABLE^{®} assembly techniques utilized by Ingenza (and described in the '679 patent, which is incorporated by reference) since the linkers are synthetic fragments of DNA which can be modified during synthesis. Once the DNA has been assembled and introduced into a microbe for replication (typically *E*. *coli),* the phosphorothioate bond will be lost due to the cells natural replication machinery only generating natural phosphodiester bonds between nucleotides.

The present invention provides at least the following advantages not previously available with current DNA assembly methods.

### 1. Automation of the inABLE^{®} Assembly.

During the process of constructing a DNA vector through inABLE^{®} assembly each part requiring assembly must go through a part-linker fusion. Automation of part-linker fusion reaction preparation is feasible through the use of a liquid handling robot. However, prior to assembly each part-linker fusion requires purification via gel electrophoresis, limiting the potential for automation. Attempts to omit the gel extraction stage result in a significant decrease in assembly efficiency likely due to the presence of contaminating fragments or vector being carried through to the assembly reaction. With the present invention, it is possible to purify the part-linker fusion without the need to run a gel. Since the exonuclease is unable to cleave phosphorothioate bonds, the part-linker fusion (POA::TP::LOA) is protected from the exonuclease, while the vector backbone is degraded.

### 2. Enhanced assembly efficiency through the removal of aberrant part-linker fusions.

The present inABLE assembly technique utilizes gel electrophoresis to separate the part-linker fusions from the vector backbone. However, on a gel it is not possible to distinguish between part-linker fusions and aberrant part-linkers (i.e., missing either POA and/or LOA). Aberrant part/linkers are incomplete or incorrect part/linkers. These fragments have an inhibitory effect on the assembly reaction and despite this are currently carried through, diminishing overall assembly efficiency. Importantly, the present invention does not rely on separation of the required DNA from contaminants but instead complete removal of the contaminating DNA.

### 3. Enhanced assembly efficiency through the protection of unligated "nicked" plasmid from cellular exonuclease prior to in vivo ligation.

The present invention does not require the addition of ligase during the assembly reaction. Attempts to include ligase in the assembly reaction results in a modest increase in assembly efficiency, but a pronounced increase in the number of incorrect assemblies. Therefore, currently *in vivo* ligation of the assembly product is utilized to repair nicks in the product. These nicks are also a potential target for cellular exonucleases. *E. coli* Exonuclease III has been shown to act at nicks in a 3' → 5' manner. The introduction of phosphorothioate bonds at the 3' end of the POl and LOl confers resistance at nicks present in the assembled vector to this exonuclease *in vivo.*

### EXAMPLE ONE

To validate the utilization of phosphorothioate containing linkers and exonuclease treatment in the place of gel electrophoresis, a two-part assembly was initially explored. The assemblies comprised of one DNA fragment containing an origin of replication and a kanamycin resistance marker, and a second fragment comprised of a tetracycline resistance marker. This allowed for assemblies to be initially verified through antibiotic resistance (selection on media containing Kan + Tet) followed by sequencing of the constructs. Part-linker fusion reactions were performed through cycling of SapI digestion and ligation of linker fragments.

Purification of part-linker fusions. The method of purification of part-linker fusions was varied in six ways, with each experiment run in triplicate. Below is a list of the part-linker fusion purification steps.
(1) The fragment of interest was isolated via gel electrophoresis followed by extraction via a QiaQuick gel extraction kit.
(2) The part-linker fusion reaction was purified using a QiaQuick PCR purification kit.
(3) No purification of the part-linker fusion was performed.
(4) Part-linker fusions prepared using phosphorothioate containing linkers treated with exonuclease followed by gel electrophoresis and extraction via a QiaQuick gel extraction kit.
(5) Part-linker fusions prepared using phosphorothioate containing linkers treated with exonuclease followed by purification using QiaQuick PCR purification kit.
(6) Part-linker fusions prepared using phosphorothioate containing linkers treated with exonuclease followed by no further purification.

Upon completion of part-linker fusion cycling, 1 µl of Exo III (10 U) and the appropriate buffer were added to reactions 4-6, and reactions were incubated at 37 °C for 30 minutes. The concentration of DNA fragments was measured and 0.05 pmol of each combined and mixed with 2 µl of NEB buffer 2, and the volume was brought to 20 µl through the addition of deionized water. The reactions were incubated at room temperature for 30 minutes. Chemically competent NEB 10β cells (NEB) were transformed with 3 µl of the assembly reaction following the manufacturer's guidelines. Dilutions of the transformation reaction were plated onto LB agar plates containing the appropriate antibiotics and the plates incubated overnight at 37 °C.

Following 16 hours incubation, colonies were counted, averaged and error calculated in order to evaluate the effect the method of purification has on the efficiency of the assembly reaction (see Graph 1 below).

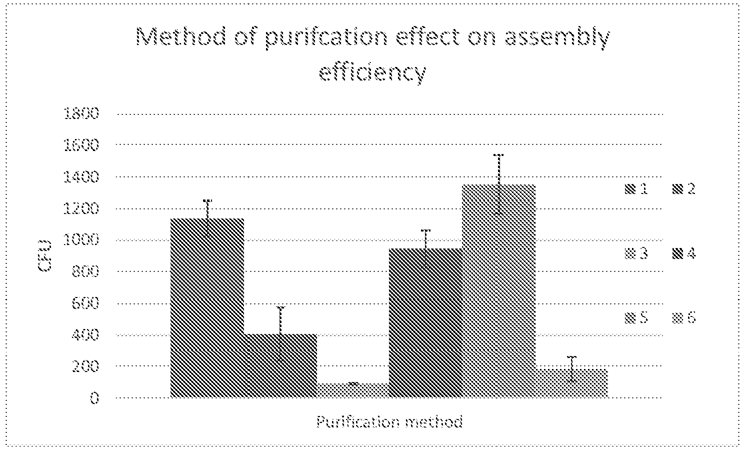

Experiments 1-3 in which standard linkers were used followed by either gel extraction, PCR purification or no purification highlights that purification via gel extraction results in the most efficient assembly reaction (bar 1). Purification using a QiaQuick PCR purification kit is a quick and automatable step which removes fragments of DNA >100 bp. However, due to the carryover of larger contaminating fragments of DNA, this has a significantly negative effect on assembly efficiency (bar 2).

In experiments 4-6, phosphorothioate containing linkers were utilized and an exonuclease treatment introduced. The combination of this with a QiaQuick PCR purification (bar 5) results in an assembly efficiency which matches that achieved through gel electrophoresis (bar 1) but removes the cumbersome and rate limiting step of running an agarose gel and extracting the DNA of interest.

Assemblies with larger number of part-linkers will also benefit from enhanced efficiency through such automation. The effect of Exonuclease III treatment plus a PCR purification (the most promising gel free work flow from the two-part assembly) on assembly efficiency was then explored to combine five DNA part-linker fusions to confirm the approach was suitable for the assembly of more complex vectors. A five-part assembly was then conducted using the protocol as described for the two-part approach with the additional parts and linkers included. The standard inABLE procedure was also performed in parallel for the five-part assembly. This experiment confirms the present invention is suitable for the assembly of larger numbers of DNA fragments without sacrificing DNA assembly efficiency as shown in FIG. 4.

### EXAMPLE TWO

### Exonuclease identification.

A number of exonucleases were identified and tested to determine which is most appropriate for polynucleic acid assembly with the present invention. SapI digestion of a truncated part results in the generation of 5' three nucleotide overhangs to which part and linker oligos are annealed generating 16 nucleotide 3' overhangs. The exonuclease of choice therefore should operate in a 3' to 5' direct and is unable to cleave phosphorothioate bonds.

Exonuclease I and III (both from *E. coli),* Exonuclease T and Bal-34 are reported to have potentially compatible characteristics with the present invention and were explored in an initial study. From this selection of 3' - 5' exonucleases, only Exonuclease III was found to remove contaminating DNA from the reaction while the phosphorothioate protected part-linker fusion was resistant to degradation.

Referring to FIG. 5, a part-linker fusion reaction was analyzed via agarose gel following exonuclease treatment. Lane 1 highlights treatment with Exonuclease III removing the contaminating DNA (higher molecular weight band seen in lanes 2, 4 and 5) while not digesting the phosphorothioate protected part-linker fusion (lower band). The other exonucleases tested either did not remove the contaminating DNA (lanes 2, 4 and 5) or digested all DNA present (lane 3).

### EXAMPLE THREE

The effect of exonuclease treatment on assembly efficiency.

Exonuclease III treatment without the use of phosphorothioate bonds was explored to determine if an exonuclease treatment alone is sufficient. A review of the characteristics of Exonuclease III suggests that it should be suitable to remove contaminating DNA from the reaction without digesting the part-linker fusion (removing the requirement for phosphorothioate bonds). This is due to its preferred substrates being blunt or recessed 3' termini with the 3' extensions over 4 bases or longer essentially being resistant to cleavage.

As described in Example 1 a two-part assembly was performed which allowed for assemblies to be verified through antibiotic resistance. Part-linker fusions were treated with Exonuclease III and either purified by agarose gel, PCR purification or not purified following exonuclease treatment. In parallel the inABLE procedure was performed as standard (no exonuclease treatment and including agarose gel-based purification). Assembly reactions and *E*. *coli* transformation were performed using the protocol described in Example 1. Dilutions of the transformation reaction were plated onto LB agar plates containing the appropriate antibiotics and the plates incubated overnight at 37 °C.

As shown in FIG. 6, treatment with exonuclease under the conditions tested above resulted in a pronounced decrease in assembly efficiency. Analysis of part-linker fusion reactions following exonuclease treatment and gel analysis confirmed that, as expected, the contaminating backbone fragment was efficiently removed and it appeared that the part/linker fusion remained undigested. However, analysis of the assembly reaction products via agarose gel suggests that when treated with Exo III the two DNA fragments do not assemble when the standard inABLE workflow is implemented. It is hypothesized this is due to partial degradation of the 16 base overhang by Exonuclease III. While this is not the preferred substrate, it is likely that this secondary reaction is occurring at a reduced rate compared to backbone degradation. To avoid this, the utilization of phosphorothioate bonds to protect these 16 base overhangs was implemented.

### EXAMPLE FOUR

The effect of phosphorothioate bonds on assembly efficiency (FIG. 7)

The introduction of a phosphorothioate bond alone may result in an increase in assembly efficiency. This is because the current process relies on *in vivo* ligation of the assembly product to repair nicks in the product. These nicks are also a potential target for cellular exonucleases. *E. coli* Exonuclease III has been characterized as being able to initiate degradation at plasmid nicks in a 3' → 5' manner. The introduction of phosphorothioate bonds at the 3' end of the POl and LOl confers resistance at nicks present in the assembled vector to this *E. coli* exonuclease *in vivo.*

To explore this, an experiment was performed in which 5 fragments of DNA were assembled. One of the fragments contained on origin of replication while the remaining four contained markers conferring resistance to kanamycin, chloramphenicol, ampicillin and tetracycline. Part-linker fusion reactions were performed using either standard linker or phosphorothioate modified linkers. All fragments were purified via the running of an agarose gel with assembly reactions and *E. coli* transformation performed as described in Example 1. Dilutions of the transformation reaction were plated onto LB agar plates containing the appropriate antibiotics and the plates incubated overnight at 37 °C. No significant difference in assembly efficiency was observed in this experiment when comparing standard to phosphorothioate containing linkers. This result, as shown in FIG. 7, suggests that the utilization of phosphorothioate linkers alone does not always provide any advantage.

### EXAMPLE FIVE

The effect of coupled phosphorothioate protection and exonuclease treatment on assembly accuracy.

In previous examples the assembly of DNA fragments conferring antibiotic resistance or containing origins of replication allowed for a direct comparison of assembly efficiency through the number of colonies present on selection plates containing the appropriate antibiotics. However, this approach does not allow for the study of assembly accuracy, i.e. the ratio of correctly assembled products to misassembles, as cells carrying misassembles will not be viable on the selection plate.

To explore assembly accuracy a four-part assembly (one fragment containing an antibiotic resistance marker and origin of replication and three comprised of a gene and associated regulatory regions) was performed and transformants screened for correctly assembled DNA vectors. Three work flows were compared, the standard inABLE procedure, phosphorothioate bonds only and coupled exonuclease treatment and phosphorothioate bonds. All fragments were purified via gel electrophoresis with assembly reactions and *E. coli* transformation performed as described in Example 1. Transformants were screened via PCR and the ratio of correctly assembled fragments to misassembles presented as assembly accuracy in FIG. 8.

In the absence of both phosphorothioate bonds and exonuclease treatment an average assembly efficiency of 2.3% was achieved. The introduction of phosphorothioate bonds into the linker sequences resulted in an increase in assembly efficiency to 26.4%, however when phosphorothioate bonds were coupled to exonuclease treatment the assembly accuracy rose to 93.3%, as shown below in Table 1.

**Table 1. Assembly Efficiency**

| | Conditions | | Experiment 1 | Experiment 2 | Standard Deviation | Average Assembly Accuracy (%) |
|---|---|---|---|---|---|---|
| | Phosphorothioate Bond Protection | Exonuclease III Treatment | Assembly Accuracy (%) | Assembly Accuracy (%) | | |
| Standard inABLE | (-) | (-) | 0 | 4.7 | 3.3 | 2.3 |
| Phosphorothioate Bond | (+) | (-) | 32.2 | 20.7 | 8.1 | 26.4 |
| Coupled Phosphorothioate Bond + Exonuclease III Treatment | (+) | (+) | 90 | 96.7 | 4.7 | 93.3 |

Certain modifications and improvements will occur to those skilled in the art upon a reading of the foregoing description. The above-mentioned examples are provided to serve the purpose of clarifying aspects of the invention and will be apparent to one skilled in the art that they do not serve to limit the scope of the invention. All modifications and improvements have been deleted herein for the sake of conciseness and readability but are properly within the scope of the following claims.

## Claims

1. A method for assembling polynucleic acids comprising:
providing at least two DNA truncated parts;
ligating a DNA modified linker having an internucleotide modification resistant to nuclease cleavage to both ends of each DNA truncated part to form at least two part-linker fusions;
isolating the part-linker fusions using an exonuclease; and
joining the isolated part-linker fusions to form a polynucleic acid sequence.

2. The method of claim 1, wherein the polynucleic acid sequence is formed by annealing the part-linker fusions.

3. The method of claim 2, further comprising ligating the polynucleic acid sequence.

4. The method of claim 1, wherein the internucleotide modification is a phosphorothioate bond.

5. The method of claim 4, wherein the internucleotide modification comprises 1 to 3 phosphorothioate bonds.

6. The method of claim 1, wherein the at least two part-linker fusions have a first part-linker fusion and a second part-linker fusion, and wherein an end of the first part-liker fusion is complementary to an end of the second part-linker fusion.

7. The method of claim 6, wherein the first part-linker fusion and the second part-linker fusion self-assemble.

8. The method of claim 1, wherein the step of isolating the part-linker fusions using an exonuclease comprises the step of removing aberrant part-linker fusions.

9. The method of claim 1, wherein the step of isolating the part-linker fusions using an exonuclease is automated.

10. The method of claim 1, wherein the step of isolating the part-linker fusions using an exonuclease does not require gel electrophoresis.

11. The method of claim 1, wherein the exonuclease comprises Exonuclease III.

12. A method for assembling polynucleic acids comprising:
providing at least two DNA truncated parts;
ligating a DNA modified linker having a phosphorothioate bond to both ends of each DNA truncated parts to form at least two part-linker fusions;
isolating the part-linker fusions using an exonuclease to remove aberrant part-linker fusions; and
joining the part-linker fusions to form a polynucleic acid sequence.

13. A method for isolating a part-linker fusion comprising:
providing a DNA truncated part;
ligating a DNA modified linker having a phosphorothioate bond to each end of the DNA truncated part to form a part-linker fusion; and
isolating the part-linker fusions using an exonuclease.

## Patentansprüche

1. Verfahren zum Zusammenbauen von Polynukleinsäuren, umfassend:
Bereitstellen von zumindest zwei DNA-beschnittenen Teilen;
Ligieren eines DNA-modifizierten Linkers, der eine Internukleotid-Modifikation aufweist, die resistent gegen Nukleasespaltung ist, an beide Enden jedes DNA-beschnittenen Teils, um zumindest zwei Teil-Linker-Fusionen zu bilden;
Isolieren der Teil-Linker-Fusionen unter Verwendung einer Exonuklease; und
Verbinden der isolierten Teil-Linker-Fusionen, um eine Polynukleinsäuresequenz zu bilden.

2. Verfahren nach Anspruch 1, wobei die Polynukleinsäuresequenz durch Annealing der Teil-Linker-Fusionen gebildet wird.

3. Verfahren nach Anspruch 2, ferner umfassend das Ligieren der Polynukleinsäuresequenz.

4. Verfahren nach Anspruch 1, wobei die Internukleotid-Modifikation eine Phosphorothioat-Bindung ist.

5. Verfahren nach Anspruch 4, wobei die Internukleotid-Modifikation 1 bis 3 Phosphorothioat-Bindungen umfasst.

6. Verfahren nach Anspruch 1, wobei die zumindest zwei Teil-Linker-Fusionen eine erste Teil-Linker-Fusion und eine zweite Teil-Linker-Fusion aufweisen und wobei ein Ende der ersten Teil-Linker-Fusion komplementär zu einem Ende der zweiten Teil-Linker-Fusion ist.

7. Verfahren nach Anspruch 6, wobei sich die erste Teil-Linker-Fusion und die zweite Teil-Linker-Fusion selbst zusammenbauen.

8. Verfahren nach Anspruch 1, wobei der Schritt des Isolierens der Teil-Linker-Fusionen unter Verwendung einer Exonuklease den Schritt des Entfernens von aberranten Teil-Linker-Fusionen umfasst.

9. Verfahren nach Anspruch 1, wobei der Schritt des Isolierens der Teil-Linker-Fusionen unter Verwendung einer Exonuklease automatisiert ist.

10. Verfahren nach Anspruch 1, wobei der Schritt des Isolierens der Teil-Linker-Fusionen unter Verwendung einer Exonuklease keine Gelelektrophorese erfordert.

11. Verfahren nach Anspruch 1, wobei die Exonuklease Exonuklease III umfasst.

12. Verfahren zum Zusammenbauen von Polynukleinsäuren, umfassend:
Bereitstellen von zumindest zwei DNA-beschnittenen Teilen;
Ligieren eines DNA-modifizierten Linkers, der eine Phosphorothioat-Bindung aufweist, an beide Enden jedes DNA-beschnittenen Teils, um zumindest zwei Teil-Linker-Fusionen zu bilden;
Isolieren der Teil-Linker-Fusionen unter Verwendung einer Exonuklease, um aberrante Teil-Linker-Fusionen zu entfernen; und
Verbinden der Teil-Linker-Fusionen, um eine Polynukleinsäuresequenz zu bilden.

13. Verfahren zum Isolieren einer Teil-Linker-Fusion, umfassend:
Bereitstellen eines DNA-beschnittenen Teils;
Ligieren eines DNA-modifizierten Linkers, der eine Phosphorothioat-Bindung aufweist, an jedes Ende des DNA-beschnittenen Teils, um eine Teil-Linker-Fusion zu bilden; und
Isolieren der Teil-Linker-Fusionen unter Verwendung einer Exonuklease.

## Revendications

1. Procédé d'assemblage d'acides polynucléiques comprenant :
la production d'au moins deux parties tronquées d'ADN ;
la ligature d'un lieur modifié par ADN possédant une modification internucléotidique résistant au clivage par nucléase aux deux extrémités de chaque partie tronquée d'ADN pour former au moins deux fusions partie-lieur ;
l'isolement des fusions partie-lieur à l'aide d'une exonucléase ; et
la jonction des fusions partie-lieur isolées pour former une séquence d'acide polynucléique.

2. Procédé selon la revendication 1, ladite séquence d'acide polynucléique étant formée par annelage des fusions partie-lieur.

3. Procédé selon la revendication 2, comprenant en outre la ligature de la séquence d'acide polynucléique.

4. Procédé selon la revendication 1, ladite modification internucléotidique étant une liaison phosphorothioate.

5. Procédé selon la revendication 4, ladite modification internucléotidique comprenant 1 à 3 liaisons phosphorothioate.

6. Procédé selon la revendication 1, lesdites au moins deux fusions partie-lieur comportant une première fusion partie-lieur et une seconde fusion partie-lieur, et une extrémité de la première fusion partie-lieur étant complémentaire à une extrémité de la seconde fusion partie-lieur.

7. Procédé selon la revendication 6, ladite première fusion partie-lieur et ladite seconde fusion partie-lieur s'auto-assemblant.

8. Procédé selon la revendication 1, ladite étape d'isolement des fusions partie-lieur à l'aide d'une exonucléase comprenant l'étape de suppression des fusions partie-lieur aberrantes.

9. Procédé selon la revendication 1, ladite étape d'isolement des fusions partie-lieur à l'aide d'une exonucléase étant automatisée.

10. Procédé selon la revendication 1, ladite étape d'isolement des fusions partie-lieur à l'aide d'une exonucléase ne nécessitant pas d'électrophorèse sur gel.

11. Procédé selon la revendication 1, ladite exonucléase comprenant l'exonucléase III.

12. Procédé d'assemblage d'acides polynucléiques comprenant :
la production d'au moins deux parties tronquées d'ADN ;
la ligature d'un lieur modifié par ADN possédant une liaison phosphorothioate aux deux extrémités de chaque partie tronquée d'ADN pour former au moins deux fusions partie-lieur ;
l'isolement des fusions partie-lieur à l'aide d'une exonucléase pour supprimer les fusions partie-lieur aberrantes ; et
la jonction des fusions partie-lieur isolées pour former une séquence d'acide polynucléique.

13. Procédé d'isolement d'une fusion partie-lieur comprenant :
la production d'une partie tronquée d'ADN ;
la ligature d'un lieur modifié par ADN possédant une liaison phosphorothioate à chaque extrémité de la partie tronquée d'ADN pour former une fusion partie-lieur ; et
l'isolement des fusions partie-lieur à l'aide d'une exonucléase.
